# NEUE EUROPÄISCHE PATENTSCHRIFT

(11) **EP 0 381 799 B2**
(45) Veröffentlichungstag und Bekanntmachung des Hinweises auf die Entscheidung über den Einspruch: **23.04.2003**
(45) Hinweis auf die Patenterteilung: 20.04.1994
(21) Anmeldenummer: 89102348.3
(22) Anmeldetag: 10.02.1989
(51) Int. Cl.: A61N 1/368, A61N 1/365

(54) **Implantierbarer Herzschrittmacher mit auf einen Maximalwert begrenzter Folgefrequenz der Stimulationen von Herzmuskelkontraktionen**
Implantable cardiac pacer with a synchrous rate of stimulation of the cardiac muscle limited to a maximal value
Stimulateur cardiaque implantable avec une fréquence synchrone de stimulation du muscle cardiaque limitée à une valeur maximale

(43) Veröffentlichungstag der Anmeldung: 16.08.1990
(73) Patentinhaber: St. Jude Medical AB, 175 84 Järfälla (SE)
(72) Erfinder: Hedin, Asa, S-113 50 Stockholm (SE); Engström, Jan, S-163 55 Spanga (SE)
(74) Vertreter: Harrison, Michael Charles

(56) Entgegenhaltungen:
- EP-A- 0 133 828
- EP-A- 0 147 820
- EP-A- 0 299 613
- US-A- 4 543 954
- Siemens Firmendruckschrift "Pulse Generator 704 - Physician's Manual", März 1985

## Beschreibung

Implantierbarer Herzschrittmacher zur Stimulation der Herztätigkeit mit auf einen Maximalwert begrenzter Folgefrequenz der Stimulationen von Herzmuskelkontraktionen Die Erfindung betrifft einen in den Körper eines Lebewesens implantierbaren Herzschrittmacher mit Mitteln zum Detektieren spontaner Herzmuskelkontraktionen im Bereich eines Atriums, mit Mitteln zum Stimulieren von Herzmuskelkontraktionen im Bereich eines Ventrikels, die nach einer Detektion einer spontanen Herzmuskelkontraktion im Bereich des Atriums eine Herzmuskelkontraktion im Bereich des Ventrikels stimulieren, und mit Mitteln zum Begrenzen der Folgefrequenz der Stimulationen von Herzmuskelkontraktionen im Bereich des Ventrikels auf einen Maximalwert.

Ein Herzschrittmacher der eingangsgenannten Art ist in der Druckschrift "Pulse Generator 704 - Physician's Manual", Siemens-Elema AB, Solna, Schweden, März 1985, beschrieben. Dieser Herzschrittmacher kann u.a. in der sogenannten DDD-Betriebsart arbeiten. In dieser Betriebsart wird nach der Detektion einer spontanen Herzmuskelkontraktion im Bereich des Atriums im Bedarfsfalle, d.h., wenn während eines sich an die Detektion der spontanen Herzmuskelkontraktion im Bereich des Atriums anschliessenden Zeitraumes, des sogenannten A-V Intervalls, keine natürliche Herzmuskelkontraktion im Bereich des Ventrikels auftritt, eine Herzmuskelkontraktion im Bereich des Ventrikels stimuliert. Ausserdem wird in dieser Betriebsart jeweils dann, wenn ein zweites Zeitintervall, das sogenannte Basis-Intervall, verstrichen ist, ohne dass eine spontane Herzmuskelkontraktion im Bereich des Atriums detektiert wurde, eine Herzmuskelkontraktion im Bereich des Atriums stimuliert, wobei die Stimulation einer Herzmuskelkontraktion im Bereich des Atriums zum einen das zweite Zeitintervall und zum anderen das A-V Intervall in Gang setzt. Dabei ist die Folgefrequenz der Stimulationen von Herzmuskelkontraktionen im Bereich des Ventrikels auf einen Maximalwert, die sogenannte Highest Synchronous Rate (HSR), in der Grössenordnung von zirka 150 Stimulationen pro Minute begrenzt.

Die beschriebene Betriebsart ist in vielen Fällen, insbesondere bei Patienten mit A-V Block, indiziert. Sie kommt jedoch nicht für Patienten in Frage, die an medikamentös nicht behandelbaren Fibrillationen des Atriums leiden. Im Falle dieser Patienten besteht nämlich die Gefahr, dass die bei der Fibrillation des Atriums auftretenden Herzmuskelkontraktionen detektiert werden, was in Anbetracht der hohen Folgefrequenz der Herzmuskelkontraktionen des Atriums im Falle einer Fibrillation dazu führen würde, dass das Herz im Bereich des Ventrikels mit einer Folgefrequenz stimuliert wird, die deren Maximalwert, also der HSR, entspricht. Im Falle von Patienten, bei denen die Gefahr von Fibrillationen des Atriums besteht, wird deshalb gewöhnlich auf eine mit VVI bezeichnete Betriebsart des Herzschrittmachers ausgewichen, in der sowohl die Detektion als auch die Stimulation von Herzmuskelkontraktionen im Bereich eines Ventrikels des Herzens erfolgt, wobei immer dann eine Herzmuskelkontraktion stimuliert wird, wenn nach einem bestimmten Zeitintervall keine natürliche Herzmuskelkontraktion detektiert wurde.

Bei einem Teil der betroffenen Patienten tritt die Fibrillation des Atriums zwar nur intermittierend auf. Dennoch muss auch bei diesen Patienten der Herzschrittmacher in der VVI-Betriebsweise arbeiten, obwohl es durchaus wünschenswert wäre, den Herzschrittmacher in der DDD-Betriebsart arbeiten zu lassen, solange keine Fibrillation des Atriums vorliegt.

Es wäre daher denkbar, Fibrillationen des Atriums zu detektieren und nur während des Auftretens von Fibrillationen des Atriums den normalerweise in der DDD-Betriebsart arbeitenden Herzschrittmacher selbsttätig auf die VVI-Betriebsart umzuschalten. Dies stösst jedoch auf technische Probleme, da es zum einen schwierig ist, das erforderliche einwandfreie intrakardiale Elektrokardiogramm bezüglich der Aktivität des Atriums zu erhalten, und es andererseits problematisch ist, einen zuverlässigen Detektions-Algorithmus zu entwikkeln, da während der Fibrillation des Atriums sehr starke Änderungen der Amplitude des intrakardiellen Elektrogramms und starke Schwankungen des Herzrythmus auftreten.

Die vorstehenden Probleme gelten in ähnliche Form auch für andere vorhofsynchrone Betriebsarten von Herzschrittmachern.

Der Erfindung liegt Aufgabe zugrunde, einen Herzschrittmacher der eingangs genannten Art so auszubilden, dass sie gefahrlos auch bei solchen Patienten anwendbar sind, die an intermittierend auftretenden Fibrillationserscheinungen des Atriums leiden.

Diese Aufgabe wird durch die Merkmale des Patentanspruchs 1 gelöst. Im Gegensatz zum Stand der Technik besitzt also im Falle des erfindungsgemässen Herzschrittmachers der der Highest Synchronous Rate (HSR) entsprechende Maximalwert, auf den die Folgefrequenz der Stimulationen des Ventrikels begrenzt ist, keinen während des Betriebs des Herzschrittmachers festen Wert, sondern einen sich in Abhängigkeit von der körperlichen Aktivität des Lebewesens ändernden Wert. Da der Maximalwert wenigstens gleich der für die jeweilige körperliche Aktivität ermittelten angepassten Herzschlagfrequenz ist, ist einerseits sichergestellt, dass das Herz des Patienten erforderlichenfalls mit einer der körperlichen Aktivität des Patienten angepassten Frequenz stimuliert werden kann, und andererseit gewährleistet, dass im Falle des Auftretens einer Fibrillation des Atriums die maximal mögliche Folgefrequenz der Stimulationen im Bereich des Ventrikels auf einen Maximalwert begrenzt wird. Dieser ist infolge des Umstandes, dass er in seinem zeitlichen Verlauf der ermittelten angepassten Herzschlagfrequenz folgt, nicht unnötig hoch, sondern orientiert sich an den physiologischen Erfordernissen. In diesem Zusammenhang ist es zweckmässig, Massnahmen zu treffen, die sicherstellen, dass der Maximalwert einen physiologisch sinnvollen oberen Grenzwert nicht übersteigen kann. Dies kann beispielsweise auf einfache Weise erreicht werden, indem die der körperlichen Aktivität angepasste Herzschlagfrequenz auf einen oberen Wert begrenzt wird, wodurch zugleich der Maximalwert der Folgefrequenz der Stimulationen im Bereich des Ventrikels begrenzt ist.

Die Druckschrift EP-A-0 147 820 zeigt einen Herzschrittmacher mit alternativer Ratenanpassung, bei dem in Abhängigkeit eines Vergleichs der atrialen Frequenz in eine Betriebsart ohne atriale Erfassung gewechselt wird, bei der eine von einem Signal eines die körperliche Aktivität erfassenden Sensors abhängige Stimulationsfrequenz eingestellt wird.

Ein Vorteil des erfindungsgemässen Herzschrittmachers besteht darin, dass die Gefahr des Auftretens von schrittmacherbedingten Tachykardien stark gemindert ist, da die Folgefrequenz der Stimulationen von Herzmuskelkontraktionen im Bereich des Ventrikels auf einen sich an der jeweiligen körperlichen Aktivität des Lebewesens orientierenden und diese nicht wesentlich übersteigenden Maximalwert begrenzt ist.

Gemäss einer Variante der Erfindung ist vorgesehen, dass der Maximalwert um einen definierten Betrag grösser als die ermittelte angepasste Herzschlagfrequenz ist. Durch diese Massnahme wird erreicht, dass auch dann, wenn die natürliche Herzschlagfrequenz die ermittelte angepasste Herzschlagfrequenz geringfügig überschreitet, noch keine Begrenzung der Folgefrequenz der Stimulationen von Herzmuskelkontraktionen im Bereich des Ventrikels erfolgt. Auf diese Weise ist sichergestellt, dass möglichst wenig in den natürlichen Rythmus des Herzens eingegriffen wird. Für die genannten Zwecke genügt es, wenn der Betrag, um den der Maximalwert grösser als die ermittelte angepasste Herzschlagfrequenz ist, 5 bis 15 Herzschläge bzw. Impulse pro Minute beträgt.

Gemäss einer Variante der Erfindung ist vorgesehen, dass die Mittel zum Stimulieren von Herzmuskelkontraktionen im Bereich des Ventrikels Stimulationen mit einer der ermittelten angepassten Herzschlagfrequenz entsprechenden Folgefrequenz vornehmen, sobald die Folgefrequenz der im Bereich des Atriums detektierten Herzmuskeikontraktionen die ermittelte angepasste Herzschlagfrequenz unterschreitet. Durch diese Massnahme ist gewährleistet, dass im Bedarfsfalle eine Stimulation des Herzens im Bereich des Ventrikels mit einer an die jeweilige körperliche Aktivität des Lebewesens angepassten Folgefrequenz erfolgt, was ohne nennenswerten technischen Aufwand möglich ist, da im Falle des erfindungsgemässen Herzschrittmachers eine der körperlichen Aktivität des Patienten angepasste Herzschlagfrequenz ohnehin ermittelt wird. In diesem Zusammenhang kann gemäss einer anderen Variante der Erfindung vorgesehen sein, dass Mittel zum Stimulieren von Herzmuskelkontraktionen im Bereich des Atriums vorgesehen sind, die bei Ausbleiben einer spontanen Herzmuskelkontraktion im Bereich des Atriums Herzmuskelkontraktionen im Bereich des Atriums mit einer der ermittelten angepassten Herzschlagfrequenz entsprechenden Folgefrequenz stimulieren, wobei die Mittel zum Stimulieren von Herzmuskelkontraktionen im Bereich des Ventrikels jeweils nach einer stimulierten Herzmuskelkontraktion im Bereich des Atriums eine Herzmuskelkontraktion im Bereich des Ventrikels stimulieren. In diesem Falle geht also jeder stimulierten Herzmuskelkontraktion im Bereich des Ventrikels entweder eine natürliche oder eine stimulierte Herzmuskelkontraktion im Bereich des Atriums voraus, wie dies physiologisch korrekt ist, wobei ausserdem die Stimulation der Herzmuskelkontraktionen sowohl im Bereich des Atriums als auch im Bereich des Ventrikels mit einer der ermittelten angepassten Herzschlagfrequenz entsprechenden

Ein Ausführungsbeispiel der Erfindung wird nachfolgend anhand der beigefügten Zeichnungen erläutert. Es zeigen:
- Fig. 1: ein Blockschaltbild eines erfindungsgemässen Herzschrittmachers, und
- Fig. 2: ein die Arbeitsweise des erfindungsgemässen Herzschrittmachers verdeutlichendes Diagramm.

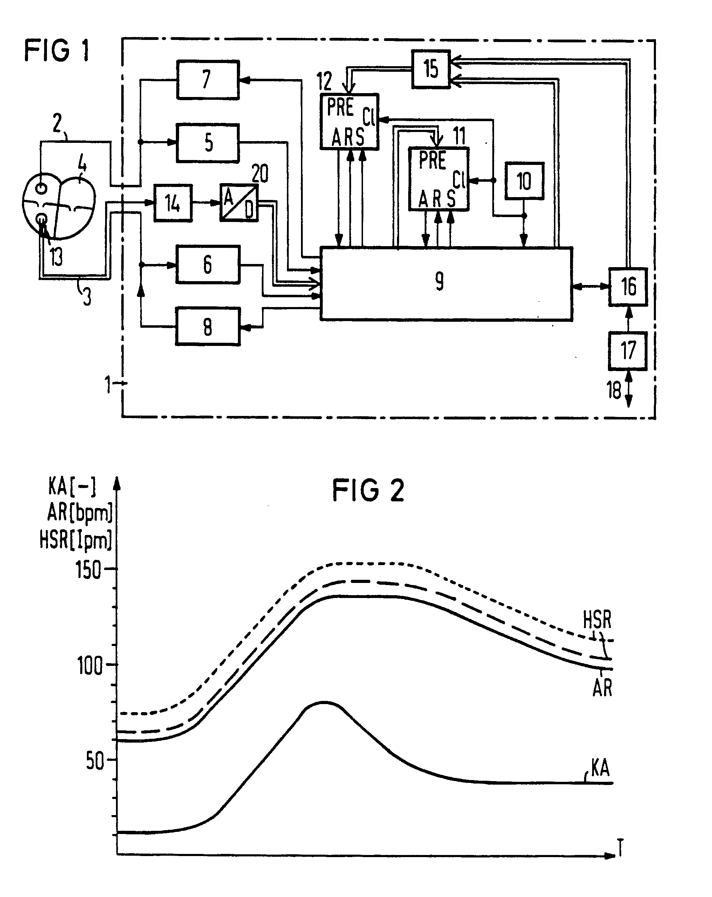

In der Fig. 1 ist ein erfindungsgemässer Herzschrittmacher dargestellt, der in der DDD-Betriebsart arbeitet. Der Herzschrittmacher, dessen Elektronik in einem hermetisch dichten, implantierbaren Gehäuse (1) angeordnet ist, steht demgemäss über zwei endokardielle Elektroden (2, 3) mit dem schematisch angedeuteten Herz (4) des den Herzschrittmacher tragenden Patienten in Verbindung. Dabei sind die Elektroden (2, 3) durch das Venensystem zu dem Herzen (4) des Patienten geführt. Die Elektrode (2) ist in dem rechten Atrium und die Elektrode (3) in dem rechten Ventrikel des Herzens (4) verankert.

Die Elektrode (2) steht mit einer Detektoreinrichtung (5) in Verbindung, die zur Detektion von spontanen Herzmuskelkontraktionen im Bereich des Atriums dient, während die Elektrode (3) mit einer Detektoreinrichtung (6) in Verbindung steht, die zur Detektion spontaner Herzmuskelkontraktionen im Bereich des Ventrikels dient. Die Elektroden (2, 3) sind ausserdem jeweils mit einem Stimulationsimpuls-Generator (7 bzw. 8) verbunden, wobei der Stimulationsimpuls-Generator (7) dazu dient, Herzmuskelkontraktionen im Bereich des Atriums zu stimulieren, während der Stimulationsimpuls-Generator (8) dazu dient Herzmuskelkontraktionen im Bereich des Ventrikels zu stimulieren.

Das Zusammenwirken der Detektoreinrichtungen (5, 6) und der Stimulationsimpuls-Generatoren (7, 8) mit dem Herz (4) wird durch eine Steuerlogik (9) gesteuert, an die ein Taktgenerator (10), ein A-V Zähler (11) und ein HSR Zähler (12) angeschlossen sind. Bei den Zählern (11, 12) handelt es sich um sogenannte PRESET-Zähler. Ein derartiger Zähler, dem Taktimpulse über einen entsprechenden Eingang C1 zugeführt sind, zählt während eines Zählvorganges jeweils eine Anzahl von Taktimpulsen ab, die durch einem mit PRE bezeichneten Eingang des Zählers zugeführte Daten bestimmt ist. Bei Erreichen der bestimmten Anzahl von Taktimpulsen gibt der Zähler an seinem Ausgang A ein entsprechendes Signal ab. Ein derartiger Zähler beginnt einen Zählvorgang, wenn einem mit S bezeichneten Eingang ein Startimpuls zugeführt wird. Der Zählvorgang wird abgebrochen und/oder der Zähler zurückgestellt, wenn einem Eingang R ein Rückstellimpuls zugeführt wird. Im Falle des beschriebenen Herzschrittmachers erhalten beide Zähler (11, 12) ihre Start- und Rückstellimpulse von der Steuerlogik (9). Ihre Taktimpulse erhalten beide Zähler (11, 12) von dem Taktgenerator (10).

Im folgenden wird die Arbeitsweise des Herzschrittmachers nur in dem Umfang beschrieben, wie dies im Zusammenhang mit der vorliegenden Erfindung erforderlich ist. Bezüglich weiterer Informationen wird auf die einschlägige Literatur, z.B. die Druckschrift "Pulse Generator 704 - Physician's Manual", Siemens-Elema AB, Solna, Schweden, März 1985, verwiesen.

Wird mittels der Detektoreinrichtung (5) eine spontane Herzmuskelkontraktion im Bereich des Atriums detektiert, gelangt von der Detektoreinrichtung (5) ein entsprechendes Signal zu der Steuerlogik (9). Diese startet daraufhin den A-V Zähler (11), der seine Taktimpulse von dem Taktgenerator (10) erhält, bei dem es sich um einen Oszillator, beispielsweise einen Quarz-Oszillator, handelt, der mit einer definierten Taktfrequenz Taktimpulse abgibt. Hat der A-V Zähler (11) eine durch seinem Eingang PRE von der Steuerlogik (9) zugeführte Daten einstellbare Anzahl von Taktimpulsen abgezählt, die der Dauer eines sogenannten A-V Intervalls entspricht, gelangt von seinem Ausgang ein entsprechendes Signal an die Steuerlogik (9). Diese aktiviert daraufhin den Stimulationsimpuls-Generator (8) zur Abgabe eines Stimulationsimpulses, der die Stimulation einer Herzmuskelkontraktion im Bereich des Ventrikels bewirkt, und stellt den A-V Zähler (11) zurück. Die Abgabe eines Stimulationsimpulses mittels des Stimulationsimpuls-Generators (8) unterbleibt jedoch, wenn während des A-V Intervalls mittels der Detektoreinrichtung (6) eine spontane Herzmuskelkontraktion im Bereich des Ventrikels detektiert wurde. Geht der Steuerlogik (9) von der Detektoreinrichtung (6) ein entsprechendes Signal zu, stellt sie den A-V Zähler (11) zurück.

Wird während eines sogenannten Basis-Intervalls, das mit der Detektion der spontanen Herzmuskelkontraktion im Bereich des Atriums zu laufen beginnt und das die Steuerlogik (9) durch Abzählen einer dessen Dauer entsprechenden Anzahl von Taktimpulsen des Taktgenerators (10) bestimmt, mittels der Detektoreinrichtung (5) keine spontane Herzmuskelkontraktion im Bereich des Atriums delektiert, aktiviert die Steuerlogik (9) am Ende des Basis-Intervalls den Stimulationsimpuls-Generator (7) zur Abgabe eines eine Herzmuskelkontraktion im Bereich des Atriums auslösenden Stimulationsimpulses. Gleichzeitig mit der Abgabe des Stimulationsimpulses startet die Steuerlogik (9) den A-V Zähler (11) wieder und beginnt ausserdem erneut mit der Bestimmung des Basis-Intervalls, wobei die Steuerlogik (9) auch am Ende des durch die Stimulation einer Herzmuskelkontraktion im Bereich des Atriums in Gang gesetzten A-V Intervalls den Stimulationsimpuls-Generator (8) zur Stimulation einer Herzmuskelkontraktion im Bereich des Ventrikels aktiviert, sofern nicht während des A-V Intervalls mittels der Detektoreinrichtung (6) eine spontane Herzmuskelkontraktion im Bereich des Ventrikels detektiert wurde.

Detektiert dagegen während des Basis-Intervalls die Detektoreinrichtung (5) eine spontane Herzmuskelkontraktion im Bereich des Atriums, führt dies in zuvor beschriebener Weise zur Stimulation einer Herzmuskelkontraktion im Bereich des Ventrikels mittels des Stimulationsimpuls-Generators (8) nach Ablauf des A-V Intervalls, es sei denn, dass während des A-V Intervalls mittels der Detektoreinrichtung (6) eine spontane Herzmuskelkontraktion im Bereich des Ventrikels detektiert wird. Ausserdem wird durch die vor dem Ablauf des Basis-Intervalls auftretende Detektion einer spontanen Herzmuskelkontraktion im Bereich des Atriums die Ermittlung des laufenden Basis-Intervalls mittels des Steuerlogik (9) unterbrochen und ein neues BasisIntervall in Gang gesetzt. Es wird somit deutlich, dass durch die Dauer des Basis-Intervalls eine untere Grenze gesetzt ist, unter die die Herzschlagfrequenz nicht absinken kann.

Eine Begrenzung der Folgefrequenz der Stimulationen von Herzmuskelkontraktionen im Bereich des Ventrikels erfolgt mittels des HSR Zählers (12). Dieser erhält seine Taktimpulse ebenfalls von dem Taktgenerator (10). Er wird von der Steuerlogik (9) jedesmal bei der Abgabe eines eine Herzmuskelkontraktion im Bereich des Ventrikels auslösenden Stimulationsimpulses zurückgesetzt und gestartet. Er zählt dann eine Anzahl von Taktimpulsen ab, die der Dauer eines Zeitintervalls entspricht, das zwischen zwei aufeinanderfolgenden Stimulationen von Herzmuskelkontraktionen im Bereich des Ventrikels wenigstens verstreichen muss. Die diesem Zeitintervall entsprechende Herzschlagfrequenz wird gewöhnlich als Highest Synchronous Rate (HSR) bezeichnet. Sobald das genannte Zeitintervall verstrichen ist, liegt am Ausgang A des HSR Zählers (12) ein entsprechendes Signal vor, solange dieser nicht zurückgestellt wird. Bei der nächsten Stimulation einer Herzmuskelkontraktion im Bereich des Ventrikels wird der HSR Zähler (12) erneut zurückgestellt und gestartet. Nur wenn das das Ende Zählvorgangs des HSR Zählers (12) bzw. des der HSR entsprechenden Zeitintervalls anzeigende Signal des HSR Zählers (12) der Steuerlogik (9) zugeführt ist, ist die Aktivierung des Stimulationsimpuls-Generators (8) möglich. Dies bedeutet, dass die Stimulation einer Herzmuskelkontraktion im Bereich des Ventrikels unterbleibt, falls ein A-V Intervall während des Zählvorganges des HSR Zählers (12) endet. Diese Stimulation wird bei Ablauf des der HSR entsprechenden Zeitintervalls jedoch nachgeholt. Es wird somit deutlich, dass unabhängig von der Folgefrequenz der im Bereich des Atriums detektierten Herzmuskelkontraktionen die Folgefrequenz der Stimulationen von Herzmuskelkontraktionen im Bereich des Ventrikels auf die HSR begrenzt ist.

Im Gegensatz zu Herzschrittmachern nach dem Stand der Technik, wo die HSR zwar programmierbar ist, jedoch während des normalen Betriebs des Herzschrittmachers einen festen Wert besitzt, ist im Falle des erfindungsgemässen Herzschrittmachers vorgesehen, dass der der HSR entsprechende Maximalwert der Folgefrequenz der Stimulationen von Herzmuskelkontraktionen im Bereich des Ventrikels in seinem zeitlichen Verlauf dem zeitlichen Verlauf einer der jeweiligen körperlichen Aktivität des den Herzschrittmacher tragenden Patienten angepassten Herzschlagfrequenz, deren Ermittlung im folgenden noch beschrieben werden wird, folgt und seinem Wert nach wenigstens gleich der ermittelten angepassten Herzschlagfrequenz ist. Durch diese Massnahme wird erreicht, dass der erfindungsgemässe Herzschrittmacher, obwohl er in der DDD-Betriebsart arbeitet, gefahrlos auch bei solchen Patienten Anwendung finden kann, die an intermittierend auftretenden Fibrillationen des Atriums leiden, da die HSR nie wesentlich höher liegt, als diejenige Herzschlagfrequenz, die der momentanen körperlichen Belastung des Patienten angepasst ist. Zugleich wird die Gefahr von schrittmacherbedingten Tachykardien erheblich verringert. Im Falle des erfindungsgemässen Herzschrittmachers ist vorgesehen, dass der Maximalwert der Folgefrequenz der Stimulationen von Herzmuskelkontraktionen im Bereich des Ventrikels nicht gleich der angepassten Herzschlagfrequenz ist, sondern um einen definierten Betrag grösser als diese ist.

Die entsprechenden Verhältnisse sind in der Fig. 2 qualitativ dargestellt. Die Fig. 2 zeigt die Verläufe der körperlichen Aktivität KA eines Patienten ohne Angabe einer Masseinheit, der dieser körperlichen Aktivität KA angepassten Herzschlagfrequenz AR in Herzschlägen pro Minute (bpm) und zwei unterschiedliche Verläufe der entsprechenden Highest Synchronous Rate HSR in Impulsen pro Minute (Ipm) über der Zeit T. Im Falle der Fig. 2 steigt die körperliche Aktivität KA des Patienten von einem relativ niedrigen Niveau aus zunächst an, um dann abzusinken und sich auf einem etwas höheren Niveau zu stabilisieren. Dieser Verlauf der körperlichen Aktivität KA spiegelt sich in dem entsprechenden Verlauf der angepassten Herzschlagfrequenz AR wieder. Dies gilt auch für den Verlauf der Highest Synchronous Rate HSR, die im einen Fall um einen definierten Betrag von 5 Herzschlägen bzw. Impulsen pro Minute (strichlierter Verlauf) und im anderen Fall um 15 Herzschläge bzw. Impulse pro Minute (punktierter Verlauf) höher als die jeweilige angepasste Herzschlagfrequenz AR liegt.

Um die der körperlichen Aktivität des Patienten angepasste Herzschlagfrequenz ermitteln zu können, was die Voraussetzung für die beschriebene Steuerung der HSR ist, ist im Falle des erfindungsgemässen Herzschrittmachers am Ende der in das rechte Ventrikel des Herzen führenden Elektrode (3) ein schematisch angedeuteter Temperatursensor (13) angebracht, der dazu dient, die Temperatur des in dem Ventrikel befindlichen venösen Blutes zu messen, die bekanntlich ein Mass für die körperliche Aktivität darstellt. Der Temperatursensor (13) steht über eine parallell zu der Elektrode (3) verlaufende Leitung mit einer Signalaufbereitungsschaltung (14) in Verbindung, von der das mittels des Temperatursensors (13) gewonnene, der körperlichen Aktivität des Patienten entsprechendes Signal zu einem Analog/Digital-Wandler (20) gelangt. Die Steuerlogik (9) ermittelt anhand der ihr zugeführten Ausgangsdaten des Analog/Digital-Wandlers (20) die der jeweiligen körperlichen Aktivität des Patienten angepasste Herzschlagfrequenz nach einem Algorithmus, wie er beispielsweise in der US-PS 45 43 954 beschrieben ist. Die Steuerlogik (9) ermittelt ausserdem diejenige Anzahl von Taktimpulsen des Taktgenerators (10) die bei dieser Herzschlagfrequenz zwischen zwei aufeinanderfolgenden Herzschlägen auftritt. Daten bezüglich dieser Anzahl von Taktimpulsen führt die Steuerlogik (9) einem Addierer (15) zu, der dazu dient, zu der genannten Anzahl von Taktimpulsen eine Anzahl von Taktimpulsen zu addieren, die demjenigen Betrag entspricht, um den die HSR die an die körperliche Aktivität des Patienten angepasste Herzschlagfrequenz überschreiten soll. Die der Summe der beiden Anzahlen von Taktimpulsen entsprechenden Ausgangsdaten des Addierer (15) sind dem Eingang PRE des HSR Zählers (12) zugeführt. Es erfolgt somit eine Begrenzung der HSR in der zuvor beschriebenen Weise.

Die der jeweils ermittelten angepassten Herzschlagfrequenz entsprechende Anzahl von Taktimpulsen angebenden Daten zieht die Steuerlogik intern als diejenigen Daten heran, die der Dauer des Basis-Intervalls entsprechen. Dies hat zur Folge, dass mittels des Stimulationsimpuls-Generators (7) Herzmuskelkontraktionen im Bereich des Atriums mit einer Folgefrequenz stimuliert werden, die der angepassten Herzschlagfrequenz entspricht, falls die Folgefrequenz der detektierten spontanen Herzmuskelkontraktionen im Bereich des Atriums unter die der körperlichen Aktivität des Patienten angepasste Herzschlagfrequenz absinkt. Dabei schliesst sich im Bedarfsfalle nach Ablauf des A-V Intervalls an eine Stimulation einer Herzmuskelkontraktion im Bereich des Atriums die Stimulation einer Herzmuskelkontraktion im Bereich des Ventrikels mittels des Stimulationsimpuls-Generators (8) an. Somit ist sichergestellt, dass die Herzschlagfrequenz des Patienten nicht unter die der jeweiligen körperlichen Aktivität des Patienten angepasste Herzschlagfrequenz absinken kann. Die angepasste Herzschlagfrequenz ist nach unten durch einen unteren Grenzwert, z.B. 60 Herzschläge pro Minute, und nach oben durch einen oberen Grenzwert, z.B. 140 Herzschläge pro Minute, auf einen physiologisch sinnvollen Bereich begrenzt.

Wie aus der Fig. 1 ersichtlich ist, besitzt der erfindungsgemässe Herzschrittmacher ausserdem ein mit der Steuerlogik (9) verbundenes Telemetrieregister (16) und einen mit diesem verbundenen Telemetrieschaltkreis (17). Der Herzschrittmacher ist also in der Lage mit einem nicht dargestellten externen Gerät, einem sogenannten Programmer, bidirektional Daten auszutauschen, was durch den Doppelpfeil (18) angedeutet ist. Es besteht somit die Möglichkeit, den Herzschrittmacher zu programmieren. Z. B. können mittels des Programmers über den Telemetrieschaltkreis (17) und das Telemetrieregister (16) unterschiedliche Werte für die Dauer des A-V Intervalls eingestellt werden, die dem Eingang PRE des wie erwähnt ebenfalls als PRESET-Zähler ausgebildeten A-V Zählers (11) von der Steuerlogik (9) über eine entsprechende Leitung zugeführt werden. Ausserdem besteht die Möglichkeit, den Wert des Betrages, um den die HSR höher liegt als die entsprechende angepasste Herzschlagfrequenz, zu verändern, indem dem entsprechenden Eingang des Addierers (15) unterschiedliche Daten bezüglich der dem jeweiligen Wert des Betrags entsprechenden Anzahl von Taktimpulsen zugeführt werden. Dies ist dadurch angedeutet, dass zwischen dem entsprechenden Eingang des Addierers (15) und dem Telemetrieregister (16) eine Leitung liegt. Weiter ist es möglich, den oberen und unteren Grenzwert der angepassten Herzschlagfrequenz zu ändern.

Obwohl die Erfindung anhand eines in der DDD-Betriebsart arbeitenden Herzschrittmachers beschrieben wurde, kann sie sinngemäss bei allen Vorhofsynchronien Herzschrittmachern Anwendung finden.

Der im Falle des Ausführungsbeispiels vorgesehene Temperatursensor (13) kann durch andere geeignete Sensoren mit entsprechend angepasster Signalaufbereitungsschaltung ersetzt werden, die ebenfalls ein der körperlichen Aktivität des Patienten entsprechendes Signal liefern.

Die beschriebene Einstellung der Dauer des Basis-Intervalls in Abhängigkeit von der körperlichen Aktivität des Patienten kann im Rahmen der Erfindung auch entfallen.

Der beschriebene spezielle Aufbau des Herzschrittmachers ist nur beispielhaft zu verstehen. Die für die Erfindung wesentlichen Funktionen können auch bei abweichenden Aufbau des Herzschrittmachers realisiert werden.

## Patentansprüche

1. In den Körper eines Lebewesens implantierbarer Herzschrittmacher mit Mitteln (5) zum Detektieren spontaner Herzmuskelkontraktionen im Bereich eines Atriums, mit Mitteln (8) zum Stimulieren von Herzmuskelkontraktionen im Bereich eines Ventrikels, die auf eine detektierte spontane Herzmuskelkontraktion im Bereich des Atriums eine Herzmuskelkontraktion im Bereich des Ventrikels stimulieren, mit Mitteln (12) zum Begrenzen der Folgefrequenz für die auf die detektierte spontane Kontraktion des Atriums folgende Stimulation des Ventrikels auf einen Maximalwert (HSR), und mit Mitteln (9, 13, 14) zur Ermittlung einer der jeweiligen körperlichen Aktivität (KA) des Lebewesens angepassten Herzschlagfrequenz (AR) wobei der Maximalwert (HSR) in seinem zeitlichen Verlauf dem der ermittelten angepassten Herzschlagfrequenz (AR) wenigstens im wesentlichen folgt und seinem Wert nach wenigstens gleich der ermittelten angepassten Herzschlagfrequenz (AR) ist.

2. Herzschrittmacher nach Anspruch 1, bei dem der Maximalwert (HSR) um einen definierten Betrag grösser als die ermittelte angepasste Herzschlagfrequenz (AR) ist.

3. Herzschrittmacher nach Anspruch 1 oder 2, bei dem die Mittel (9, 13, 14) zur Ermittlung einer der jeweiligen körperlichen Aktivität (KA) des Lebewesens angepassten Herzschlagfrequenz (AR) eine Sensoreinrichtung (13, 14) aufweisen, die ein der körperlichen Aktivität des Lebewesens entsprechendes Signal bildet, anhand dessen die Ermittlung der angepassten Herzschlagfrequenz (AR) erfolgt.

4. Herzschrittmacher nach einem der Ansprüche 1 bis 3, bei dem die Mittel (8) zum Stimulieren von Herzmuskelkontraktionen im Bereich des Ventrikels Stimulationen mit einer der ermittelten angepassten Herzschlagfrequenz (AR) entsprechenden Folgefrequenz vornehmen, sobald die Folgefrequenz der im Bereich des Atriums detektierten Herzmuskelkontraktionen die ermittelte angepasste Herzschlagfrequenz (AR) unterschreitet.

5. Herzschrittmacher nach einem der Ansprüche 1 bis 3, bei dem Mittel (7) zum Stimulieren von Herzmuskelkontraktionen im Bereich des Atriums vorgesehen sind, die bei Ausbleiben einer spontanen Hezmuskelkontraktion im Bereich des Atriums Herzmuskelkontraktionen im Bereich des Atriums mit einer der ermittelten angepassten Herzschlagfrequenz (AR) entsprechenden Folgefrequenz stimulieren, wobei die Mittel (8) zum Stimulieren von Herzmuskelkontraktionen im Bereich des Ventrikels im Bedarfsfall nach einer stimulierten Herzmuskelkontraktion im Bereich des Atriums eine Herzmuskelkontraktion im Bereich des Ventrikels stimulieren.

## Claims

1. Cardiac pacemaker which is implantable into the body of a living organism and which has means (5) for the detection of spontaneous cardiac muscle contractions in the region of an atrium and which has means (8) for the stimulation of cardiac muscle contractions in the region of a ventricle, which means, in response to a detected spontaneous cardiac muscle contraction in the region of the atrium, stimulate a cardiac muscle contraction in the region of the ventricle, and which has means (12) for limiting the succession frequency for the following stimulation of the ventricle, in response to the detected spontaneous contraction of the atrium, to a maximum value (HSR), and with means (9, 13, 14) for the determination of a heart rate (AR) adapted to the respective physical activity (KA) of the living organism, whereby the maximum value (HSR), in its temporal progression, at least substantially follows that of the determined adapted heart rate (AR) and, in terms of its value, is at least equal to the determined adapted heart rate (AR).

2. Cardiac pacemaker according to claim 1, in which the maximum value (HSR) is greater, by a defined amount, than the determined adapted heart rate (AR).

3. Cardiac pacemaker according to claim 1 or 2, in which the means (9, 13, 14) for the determination of a heart rate (AR) adapted to the respective physical activity (KA) of the living organism exhibit a sensor device (13, 14) which forms a signal which corresponds to the physical activity of the living organism and with the aid of which the determination of the adapted heart rate (AR) takes place.

4. Cardiac pacemaker according to one of claims 1 to 3, in which the means (8) for the stimulation of cardiac muscle contractions in the region of the ventricle undertake stimulations with a succession frequency corresponding to the determined adapted heart rate (AR), as soon as the succession frequency of the cardiac muscle contractions detected in the region of the atrium falls below the determined adapted heart rate (AR).

5. Cardiac pacemaker according to one of claims 1 to 3, in which means (7) are provided for the stimulation of cardiac muscle contractions in the region of the atrium, which means, in the absence of a spontaneous cardiac muscle contraction in the region of the atrium, stimulate cardiac muscle contractions in the region of the atrium with a succession frequency corresponding to the determined adapted heart rate (AR), in which case the means (8) for the stimulation of cardiac muscle contractions in the region of the ventricle, if required, after a stimulated cardiac muscle contraction in the region of the atrium, stimulate a cardiac muscle contraction in the region of the ventricle.

## Revendications

1. Stimulateur cardiaque implantable dans le corps d'un être vivant, comportant des moyens (5) pour détecter des contractions spontanées du muscle cardiaque dans la zone d'une oreillette, des moyens (8) pour stimuler des contractions du muscle cardiaque au niveau d'un ventricule et qui, sur une contraction spontanée détectée du muscle cardiaque au niveau de l'oreillette, stimulent une contraction du muscle cardiaque au niveau du ventricule, des moyens (12) pour limiter la fréquence de récurrence de la stimulation du ventricule suivant la contraction spontanée détectée de l'oreillette, à une valeur maximale (HSR), et des moyens (9, 13, 14) pour déterminer une fréquence adaptée déterminée (AR) des battements cardiaques à l'activité corporelle (kA) de l'être vivant, la valeur maximale (HSR), ayant dans le temps qui suit au moins pour l'essentiel l'allure dans le temps de la fréquence adaptée déterminée (AR) des battements cardiaques et étant au moins égale à la fréquence adaptée déterminée (AR) des battements cardiaques.

2. Stimulateur cardiaque suivant la revendication 1, dans lequel la valeur maximale (HSR)est supérieure, d'une valeur définie, à la fréquence adaptée déterminée (AR) des battements cardiaques.

3. Stimulateur cardiaque suivant la revendication 1 ou 2, dans lequel les moyens (9, 13, 14) pour déterminer une fréquence (AR) des battements cardiaques, adaptée à l'activité corporelle respective (KA) de l'être vivant, possèdent un dispositif à capteurs (13, 14), qui forme un signal qui correspond à l'activité corporelle de l'être vivant et sur la base duquel s'effectue la détermination de la fréquence adaptée (AR) des battements cardiaques.

4. Stimulateur cardiaque suivant l'une des revendications 1 à 3, dans lequel, les moyens (8) servant à stimuler des contractions du muscle cardiaque au niveau du ventricule réalisent des stimulations avec une fréquence de récurrence qui correspond à la fréquence adaptée déterminée (AR) des battements cardiaques, dès que la fréquence de récurrence des contractions du muscle cardiaque détectées au niveau de l'oreillette tombe au-dessous de la fréquence adaptée déterminée (AR) des battements cardiaques.

5. Stimulateur cardiaque suivant l'une des revendications 1 à 3, dans lequel il est prévu des moyens (7) pour stimuler des contractions du muscle cardiaque dans la zone de l'oreillette, qui lorsqu'il n'y a plus une contraction spontanée du muscle cardiaque dans la zone de l'oreillette stimulent des contractions du muscle cardiaque au niveau de l'oreillette avec une fréquence de récurrence qui correspond à la fréquence adaptée déterminée (AR) des battements cardiaques, les moyens (8) servant à stimuler des contractions du muscle cardiaque au niveau du ventricule stimulant, le cas échéant, après une contraction stimulée du muscle cardiaque au niveau de l'oreillette, une contraction du muscle cardiaque au niveau du ventricule.
